# FASCICULE DE BREVET EUROPEEN

(11) **EP 2 938 594 B1**
(45) Date de publication et mention de la délivrance du brevet: **17.06.2020**
(21) Numéro de dépôt: 13821883.9
(22) Date de dépôt: 20.12.2013
(51) Int. Cl.: C07C 68/06, C07C 69/96, C07D 317/36, C08G 64/30

(54) **PROCÉDÉ DE SYNTHÈSE D'ESTERS CARBONIQUES LINÉAIRES DE GLYCÉROL ALPHA/ALPHA PRIME-ALCOXYLÉS**
VERFAHREN ZUR SYNTHESE VON ALPHA/ALPHA-PRIME-ALCOXYLIERTEN LINEAREN GLYCEROLCARBONESTERN
METHOD FOR THE SYNTHESIS OF ALPHA/ALPHA-PRIME-ALCOXYLATED GLYCEROL LINEAR CARBONIC ESTERS

(30) Priorité: 26.12.2012 FR 1262796
(43) Date de publication de la demande: 04.11.2015
(73) Titulaire: Chryso, 92440 Issy-Les-Moulineaux (FR); Institut National Polytechnique de Toulouse, 31400 Toulouse (FR); Institut National de la Recherche Agronomique (INRA), 75007 Paris (FR)
(72) Inventeur: LEISING, Frédéric, F-60300 Avilly Saint-Leonard (FR); MOULOUNGUI, Zéphirin, F-31200 Toulouse (FR)
(74) Mandataire: Lavoix
(86) Numéro de dépôt international: PCT/FR2013/053225
(87) Numéro de publication internationale: WO 2014/102495

(56) Documents cités:
- EP-A1- 2 151 465
- WO-A2-2008/074450
- FR-A1- 2 874 217
- US-A1- 2004 198 991
- HU Y ET AL: "3-Deoxy-3-substituted-d-myo-inositol imidazolyl ether lipid phosphates and carbonate as inhibitors of the phosphatidylinositol 3-kinase pathway and cancer cell growth", BIOORGANIC & MEDICINAL CHEMISTRY LETTERS, PERGAMON, vol. 11, no. 2, 1 janvier 2001 (2001-01-01), pages 173-176, XP004314841, ISSN: 0960-894X, DOI: 10.1016/S0960-894X(00)00640-5 cité dans la demande
- EZHOVA N N ET AL: "Glycerol carboxylation to glycerol carbonate in the presence of rhodium complexes with nitrogen-containing macroligands", PETROLEUM CHEMISTRY, NAUKA/INTERPERIODICA, MO, vol. 52, no. 6, 6 novembre 2012 (2012-11-06), pages 416-421, XP035134264, ISSN: 1555-6239, DOI: 10.1134/S0965544112060060 cité dans la demande
- DATABASE BEILSTEIN BEILSTEIN INSTITUTE FOR ORGANIC CHEMISTRY, FRANKFURT-MAIN, DE; 1994, "RX-ID: 4173540", XP002714150, cité dans la demande

## Description

L'invention concerne un procédé de synthèse de nouveaux composés, dits esters carboniques linéaires de glycérol α/α'-alcoxylés (alpha/alpha prime-alcoxylés) -notamment sous forme d'oligomères d'esters carboniques linéaires de glycérol α/α'-alcoxylés-. L'invention concerne aussi les nouveaux composés ainsi obtenus, une composition comprenant au moins un tel nouvel ester carbonique linéaire de glycérol α/α'-alcoxylés, ainsi que les utilisations de ces composés.

On sait que des polymères de haut poids moléculaires non alcoxylés peuvent être produits à partir de carbonates cycliques à 6-chaînons en présence d'initiateurs anioniques tels que des alcoxydes métalliques (Li⁺, K, carbonate alcalins, alkyles métalliques, alcoolates métalliques).

On connaît un procédé de polymérisation cationique d'un carbonate cyclique à 6 chaînons porteur d'un groupe allylique pendant lié au cycle carbonate par un pont éther. Pour contenir un pont éther pendant, le monomère doit être au préalable O-éthérifié afin de conserver le groupement éther O-allylique dans le polycarbonate résultant. La publication « Synthesis, characterization and ring-opening polymerization of a novel six-membered cyclic carbonate bearing pendent allyl ether group » He F. et al., (2008), Polymer, 49, 1185-1190 décrit un procédé de polymérisation d'un monomère cyclo-carbonate de glycérol à 6 chaînons porteur d'un éther allylique, qui conduit à des polymères de carbonate de glycérol β-allyloxylés. Ce procédé ne permet pas l'obtention de carbonates linéaires de glycérol α/α'-alcoxylés. En particulier, si l'ouverture d'un cycle à 6 chaînons est relativement aisée, tel n'est pas le cas d'un cycle à 5 chaînons.

On connaît :
- de EP 2 151 465 un procédé catalytique de polymérisation de carbonate de triméthylène issu de ressources renouvelables ;
- de Nedlova NA et al. (1994), Russian Journal of Organic Chemistry, 30, 599-603 et de Ezhova et al. ; (2012), Petroleum chemistry, 50(20), 416-421, des procédés de synthèse de composés uréthane ;
- de WO 2008/074450 des composes nitrés inhibiteurs non peptidiques de la rénine ;
- de Hu Y et al., (2001), Bioorganic & Medicinal Chemistry Letters, 11, 173-176 et de US 2004/0198991 des carbonates linéaires de glycérol alcoxylés ;
- de FR 2 874 217 une composition comprenant notamment des polyglycérols de formule suivante :

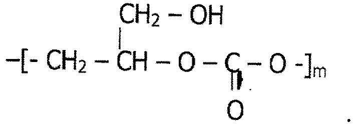

La revue générale « Aliphatic cyclic carbonates and spiroorthocarbonates as monomers », Rokicki G., (2000), Prog. Polym. Sci., 25, pp 259-342 rapporte la polymérisation du carbonate d'éthylène et du carbonate de propylène en présence d'un catalyseur de transestérification, à une température respectivement de 170°C et 180°C. Elle indique que la polymérisation des carbonates cycliques de glycérol à 5 chaînons pour former des composés poly(éthylène carbonate) ou poly(propylène carbonate) est thermodynamiquement défavorable et que cette polymérisation s'accompagne d'une décarboxylation importante et d'un dégagement de CO₂ corrélé avec la formation de poly(éther-carbonate)s présentant une proportion de fonction carbonate au plus égale à 50 % en mole. Une telle polymérisation nécessite en outre des produits de départ d'origine pétrochimique (carbonate d'éthylène et du carbonate de propylène), une durée de réaction très longue -notamment de plusieurs jours-, et est incompatible avec son exploitation à l'échelle industrielle. En outre, un tel procédé ne conduit qu'à des oligomères de faible poids moléculaire.

Il n'était donc jusqu'à maintenant pas possible d'obtenir des esters carboniques linéaires de glycérol α/α'-alcoxylés, notamment sous formes d'oligomères, en moins de 24 heures et dans des conditions compatibles avec une exploitation à l'échelle industrielle.

L'invention vise à pallier ces inconvénients.

L'invention vise donc à proposer un procédé permettant d'obtenir des nouveaux composés, dits esters carboniques linéaires de glycérol α/α'-alcoxylés comprenant au moins un motif résultant de l'ouverture du cycle à 5 chaînons d'un cyclocarbonate de glycérol dans lequel l'un au moins des carbones α/α' du groupement issu du glycérol forme une liaison éther-oxyde covalente avec un groupement organique constituant, vis-à-vis du motif issu du carbonate de glycérol, une chaine latérale du type « alcoxy » (éther-oxyde).

L'invention vise aussi en particulier à proposer un tel procédé susceptible d'être mis en œuvre dans des conditions compatibles avec une exploitation à l'échelle industrielle, notamment à température modérée et avec des faibles durées de réaction - en particulier en moins de 24 heures -, de façon simple et peu coûteuse, à partir de réactifs issus de ressources naturelles -notamment de ressources végétales- qui sont renouvelables.

L'invention vise aussi en particulier à proposer un procédé permettant d'obtenir une composition organique d'esters carboniques linéaires de glycérol α/α'-alcoxylés qui est directement exploitable.

L'invention vise aussi à proposer un tel procédé permettant l'insertion de groupements fonctionnels hétéroatomiques -notamment de groupements aminés ou oxygénés- dans des esters carboniques linéaires de glycérol α/α'-alcoxylés, notamment sous forme d'oligomères, lors de leur synthèse.

L'invention vise aussi à proposer un tel procédé permettant la synthèse de nouveaux esters carboniques linéaires de glycérol alcoxylés - notamment sous forme d'oligomères- de masse moléculaire contrôlée pouvant être variable. En particulier, l'invention vise aussi à proposer un tel procédé qui permette la synthèse de nouveaux esters carboniques linéaires de glycérol alcoxylés -notamment sous forme d'oligomères- présentant une masse molaire supérieure à 500 g/mole, notamment supérieur à 1000 g/mole.

En particulier, l'invention vise à proposer un tel procédé de synthèse qui permette la valorisation d'une ressource végétale telle que le glycérol qui constitue un coproduit abondant et disponible issu de la fabrication de biodiesels. L'invention vise aussi à proposer un tel procédé qui ne nécessite pas l'utilisation d'un cyclo-carbonate d'origine pétrochimique (tel que l'éthyloxy-cyclocarbonate de glycérol ou le propyloxy-cyclocarbonate de glycérol). L'invention vise aussi à proposer un tel procédé de synthèse qui ne nécessite pas l'utilisation d'un solvant hydrocarboné issu d'une ressource fossile, toxique pour l'environnement et pour la santé humaine et non renouvelable.

L'invention vise aussi à proposer des oligomères d'esters carboniques linéaires de glycérol alcoxylés

L'invention vise en particulier à proposer des oligomères d'esters carboniques linéaires de glycérol alcoxylés présentant d'une part une résistance chimique -notamment une résistance à l'hydrolyse- qui est améliorée, mais qui d'autre part sont biodégradables en particulier par voie enzymatique.

L'invention vise aussi en particulier à proposer des oligomères d'esters carboniques linéaires de glycérol alcoxylés permettant de moduler les propriétés rhéologiques d'une composition les contenant.

En particulier, l'invention vise aussi à proposer des oligomères d'esters carboniques linéaires de glycérol alcoxylés présentant au moins une chaine latérale organique de polarité sensiblement proche de la polarité d'une liaison C-C entre deux atomes de carbone sp3.

L'invention vise aussi en particulier à proposer des oligomères d'esters carboniques linéaires de glycérol alcoxylés de faible polarité mais présentant cependant des extrémités terminales de chaine principale adaptées pour augmenter leur affinité avec une surface -notamment une surface métallique-.

L'invention vise aussi en particulier à proposer des oligomères d'esters carboniques linéaires de glycérol alcoxylés susceptibles de pouvoir être obtenus à partir de ressources naturelles et renouvelables et qui peuvent être qualifiés de « biosourcés ».

Dans tout le texte, on désigne par :
- « carbone a» et/ou « carbone a' » d'un dérivé du glycérol, les deux atomes de carbone qui sont porteurs de deux atomes d'hydrogène et par « carbone β » d'un dérivé du glycérol, l'atome de carbone qui est porteur d'un seul atome d'hydrogène ;
- « ester carbonique linéaire de glycérol » : tout composé susceptible d'être obtenu par ouverture du cycle carbonate d'un cyclo-carbonate de glycérol ;
- « chaine principale » d'un ester carbonique linéaire de glycérol alcoxylé, l'ensemble de cardinal le plus élevé des atomes de l'ester carbonique linéaire de glycérol alcoxylé qui sont linéairement liés les uns aux autres et comprenant au moins l'un des atomes de carbone α et/ou α' du glycérol et les atomes de carbone β du glycérol ;
- « chaine latérale » d'un ester carbonique linéaire de glycérol alcoxylé, l'ensemble d'atomes linéairement liés les uns aux autres par les liaisons covalentes, ledit ensemble d'atomes étant distinct de la chaine principale et lié à ladite chaine principale par un groupement éther-oxyde -CH₂-O-.

L'invention concerne donc un procédé de synthèse de nouveaux esters carboniques linéaires de glycérol α/α'-alcoxylés -notamment sous forme d'oligomères- dans lequel on met en contact :
i. une quantité d'au moins un précurseur choisi dans le groupe formé des cyclo-carbonates de glycérol α/α'-alcoxylés de formule (XXII) suivante : 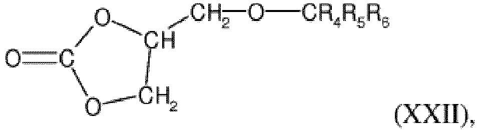 dans laquelle
   - R₄, R₅ et R₆ sont des hydrogènes, ou
   - R₄ et R₅ sont des hydrogènes et R₆ est un groupement alkyle ou amino-alkyle présentant de 1 à 5 atomes de carbone, ou
   - R₄, R₅ et R₆ sont choisis dans le groupe formé de l'hydrogène et des groupements organiques de formule générale (VII) suivante : 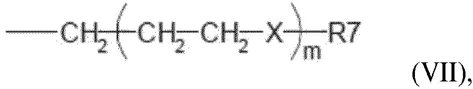 dans laquelle :
      ▪ R₇ est choisi dans le groupe formé d'un H et d'un groupement hydrocarboné présentant de 1 à 6 atomes de carbone ;
      ▪ m est un nombre entier compris entre 0 et 10 bornes incluses, et ;
      ▪ X est choisi parmi -O- et -NH-, et ;
ii. une quantité d'au moins un catalyseur choisi dans le groupe formé des oxydes métalliques, des alcoxydes métalliques, des acides de Lewis, des catalyseurs organo-métalliques et des bases minérales ;
iii. une quantité d'au moins un amorceur organique choisi dans le groupe formé des alcools, des polyols et des amino-alcools ;
à une température de réaction inférieure à 220°C, notamment comprise entre 100°C et 220°C, en particulier comprise entre 150°C et 220°C -plus particulièrement comprise entre 140°C et 200°C, de préférence sensiblement de l'ordre de 180°C-caractérisé en ce que dans une première étape, on mélange et on chauffe le(s) précurseur(s), le(s) catalyseur(s) et l'(les) amorceur(s) organique(s) dans un réacteur clos étanche aux gaz de façon à atteindre la température de réaction et une pression, dite pression autogène, supérieure à la pression atmosphérique dans le réacteur clos, puis, à la température de réaction, dans une deuxième étape, on réalise une fuite de composition gazeuse de façon à diminuer la pression dans le réacteur et on maintient la température de réaction dans le réacteur pendant une durée supérieure à 5 minutes.

L'invention concerne donc un procédé de synthèse de nouveaux esters carboniques linéaires de glycérol α/α'-alcoxylés -notamment sous forme d'un oligomère d'ester carbonique linéaire de glycérol α/α'-alcoxylé- par ouverture catalytique du cycle à cinq chaînons d'un précurseur cyclo-carbonate de glycérol α/α'-alcoxylé et oligomérisation en présence d'un catalyseur -par exemple d'un catalyseur de Lewis ou de transestérification- et d'un amorceur organique qui peut être monofonctionnel ou polyfonctionnel.

De façon totalement inattendue, il s'avère que le traitement d'un précurseur choisi dans le groupe formé des cyclo-carbonates de glycérol α/α'-alcoxylés de formule (XXII) en présence à la fois d'un catalyseur et d'un amorceur organique, suffit pour permettre l'ouverture du cycle du cyclo-carbonate à cinq chaînons à une température de réaction inférieure à 220°C, et l'obtention d'esters carboniques linéaires de glycérol α/α'-alcoxylés -notamment sous forme d'oligomères- sans perte totale des groupements carbonates. Une explication possible de ce phénomène surprenant serait que le catalyseur permettrait l'ouverture catalytique du cycle d'un premier précurseur par l'amorceur organique, puis de façon synergique une attaque de l'amorce ainsi formée sur un deuxième précurseur conduisant à une oligomérisation :
- soit par une attaque nucléophile privilégiée d'un hydroxyle de l'amorceur organique sur le carbone du carbonyle du précurseur cyclo-carbonate de glycérol α/α'-alcoxylé de formule (XXII) conduisant à la formation d'un ester carbonique linéaire de glycérol α/α'-alcoxylé,
- soit par une attaque nucléophile minoritaire d'un hydroxyle de l'amorceur organique sur l'un des atomes de carbone du cycle du précurseur cyclo-carbonate de glycérol α/α'-alcoxylé de formule (XXII) conduisant à la formation irréversible d'un groupement éther par décarbonatation spontanée et à la libération d'un groupement hydroxyle.

Les inventeurs ont découvert qu'il est possible de réaliser la synthèse d'un tel ester carbonique linéaire de glycérol α/α'-alcoxylé dans une durée réduite -notamment de l'ordre de 2 heures- en mettant en contact dans un réacteur un précurseur cyclo-carbonate de glycérol α/α'-alcoxylé de formule (XXII), un catalyseur et un amorceur organique à une température de réaction inférieure à 220°C et sous pression, dite pression autogène, générée par le chauffage du mélange réactionnel dans le réacteur hermétiquement clos.

Avantageusement, lorsque le mélange atteint la température de réaction, on ouvre le réacteur et on équilibre la pression interne du réacteur avec la pression atmosphérique.

Les inventeurs ont observé que la mise en surpression du réacteur contenant le mélange forme de chaque précurseur, de chaque catalyseur et de chaque amorceur organique ne provoque pas la décomposition du précurseur en glycérol α/α'-alcoxylé et en dioxyde de carbone, mais permet au contraire une ouverture du cycle du cyclo-carbonate précurseurs et une oligomérisation du précurseur induite par l'amorceur organique et la formation d'un nouvel ester carbonique linéaire de glycérol α/α'-alcoxylé.

Dans un procède selon l'invention, on chauffe la composition formée dans le réacteur hermétiquement clos jusqu'à une température de réaction inférieure à 220°C. Le chauffage de la composition dans le réacteur hermétiquement clos génère une pression, dite pression autogène. Lorsque la température de réaction est atteinte, on réalise une fuite de composition gazeuse dans le réacteur de façon à diminuer la pression dans le réacteur. On favorise alors la formation d'esters carboniques linéaires de glycérol α/α'-alcoxylés -notamment sous forme d'oligomères-répondant à la formule (VI) indiquée ci-après et dans laquelle x est égal à 1.

Avantageusement et selon l'invention, lorsque le mélange formé atteint la température de réaction, on ouvre le réacteur de façon à diminuer la pression interne du réacteur. Pour ce faire :
- on réalise une fuite de composition gazeuse du réacteur de façon à ajuster la pression dudit réacteur à une valeur de pression comprise entre la valeur de la pression atmosphérique et une valeur de pression, dite pression autogène, atteinte dans le réacteur clos à la température de réaction, et ;
- on maintient la température de réaction pendant une durée adaptée pour permettre une polymérisation d'au moins une partie de la quantité de précurseur cyclo-carbonate de glycérol α/α'-alcoxylé de formule (XXII) et la formation d'oligomères d'esters carboniques linéaires de glycérol α/α'-alcoxylés.

Lorsque le mélange réactionnel a atteint la température de réaction, on débouche une soupape de décompression du réacteur de façon à placer le mélange réactionnel sensiblement à la pression atmosphérique et on maintient le mélange réactionnel à la température de réaction.

Avantageusement et selon l'invention, on maintient le chauffage de la composition dans le réacteur pendant une durée de réaction comprise entre 1 heure et 24 heures à la pression atmosphérique, en particulier comprise entre 1 heure et 6 heures, de préférence de l'ordre de 2 heures.

Avantageusement, on réalise la synthèse dans un réacteur étanche aux gaz de manière à contrôler la décarboxylation et la rétention de CO₂ dans l'oligomère par la maitrise de la température plafond de réaction (par exemple 170°C) et de la pression de plafond (de l'ordre de 3 bar). L'oligomère alterne dans la chaine principale des groupements propylène carbonate linéaires et des groupements propylène porteurs de motifs méthyle O-alcoxylés formant des chaines latérales. La conservation du CO₂ dans l'oligomère est de l'ordre 30-50 mol/%.

Il s'avère de façon totalement surprenante que le traitement d'un précurseur choisi dans le groupe formé des cyclo-carbonates de glycérol α/α'-alcoxylés de formule (XXII) en présence à la fois d'un catalyseur et d'un amorceur organique, et dans lequel on module la pression autogène à l'intérieur du réacteur conduit préférentiellement sélectivement à un hétérooligomère comprenant des motifs d'ester carbonique linéaire de glycérol α/α'-alcoxylés (x = 1 dans la formule (VI) indiquée ci-après) et des motifs de glycérol α/α'-alcoxylé (x = 0 dans la formule (VI) indiquée ci-après).

Avantageusement, lors de la deuxième étape on maintient la température de réaction pendant une durée comprise entre 5 minutes et 6 heures.

Avantageusement, l'amorceur organique est un amorceur organique nucléophile adapté pour permettre une attaque nucléophile du précurseur et la formation d'oligomères d'esters carboniques linéaires de glycérol α/α'-alcoxylés présentant une extrémité terminale fonctionnalisée -notamment extrémité terminale aminée-.

Avantageusement et selon l'invention, au moins un amorceur organique est un polyol. Avantageusement et selon l'invention, au moins un amorceur organique est le glycérol. Avantageusement, le choix du glycérol comme amorceur organique permet d'obtenir un ester carbonique linéaire de glycérol α/α'-alcoxylé qui est « biosourcé ».

Avantageusement et selon l'invention, l'amorceur organique est choisi dans le groupe formé des groupements organiques aliphatiques, c'est-à-dire ne présentant pas de groupement aromatique (hydrocarboné ou hétérocyclique).

Avantageusement et selon l'invention, au moins un amorceur organique est choisi dans le groupe formé des amino-alcools présentant au moins une amine choisie dans le groupe formé des amines primaires, des amines secondaires et des amines tertiaires.

Avantageusement et selon l'invention, au moins un amorceur organique est la triéthanolamine. On choisit donc la triéthanolamine à titre d'amorceur organique comprenant une amine tertiaire.

Avantageusement et selon l'invention, au moins un amorceur organique est choisi dans le groupe formé des amino-alcools présentant une chaine principale comprenant de 2 à 10 atomes de carbone -notamment NH₂-(CH₂)_{z}-OH, dans laquelle z est un nombre entier compris entre 2 et 10, par exemple l'éthanolamine (NH₂-CH₂-CH₂-OH)-.

Avantageusement et selon l'invention, au moins un amorceur organique est choisi dans le groupe formé du 1-amino-1-cyclopentaneméthanol, du 4-aminocyclohexanol, de l'amino-1-butanol, du 2- amino-1-butanol, du 2- amino-1-phényl-1,3-propanediol, du 3- amino-3-phénylpropan-1-ol et du 2- amino-3-phényl-1-propan-1-ol.

Avantageusement, l'amorceur organique est adapté pour pouvoir réagir avec le précurseur dans le milieu de réaction liquide à la température de réaction. En particulier, avantageusement et selon l'invention, l'amorceur organique présente une température d'ébullition suffisamment élevée et adaptée pour permettre une réaction d'amorçage avec le précurseur à la température de réaction.

Le choix du précurseur α/α'-alcoxylé et de l'amorceur organique mis en présence du catalyseur sous pression autogène permet d'obtenir un ester carbonique linéaire de glycérol α/α'-alcoxylé, notamment un oligomère d'ester carbonique linéaire de glycérol α/α'-alcoxylé présentant une proportion molaire de groupement ester carbonique de glycérol par rapport au nombre total de monomère compris entre 30% et 50%.

La combinaison de la température plafond de réaction inférieure à 220°C et une pression supérieure ou égale à la pression atmosphérique, notamment une pression comprise entre 1000 et 5000 hPa, en particulier de l'ordre de 3000 hPa sont des conditions de l'ouverture du cycle du précurseur cyclo-carbonate de glycérol α/α'-alcoxylé et de conservation au moins partielle de groupement carbonate dans l'oligomère ester carbonique linéaire de glycérol α/α'-alcoxylé. Le tandem précurseur /amorceur organique augure la mise en contact du précurseur avec l'amorceur organique. Le catalyseur complète l'ensemble du système réactionnel.

Avantageusement et selon l'invention, on choisit un catalyseur dans le groupe formé des sels métalliques -notamment des acides de Lewis du type sulfate métallique, en particulier du sulfate de zinc (ZnSO₄)- des sulfates supportés sur résine cationique et des sels organométalliques, par exemple des carboxylates de zinc -notamment du stéarate de zinc (CH₃-(CH₂)₁₆-COO)₂Zn)-, des carboxylates d'étain -notamment du stéarate d'étain- et des carboxylates de zirconium -notamment du stéarate de zirconium -. Un tel sel métallique à titre de catalyseur est apte à former un complexe entre le métal du catalyseur et l'atome d'oxygène du carbonyle du précurseur.

Avantageusement et selon l'invention, au moins un catalyseur est choisi dans le groupe formé du sulfate de zinc et du stéarate de zinc.

En variante, avantageusement et selon l'invention, on choisit un catalyseur dans le groupe formé des oxydes métalliques -par exemple l'oxyde de zirconium (ZrO₂) et l'oxyde de titane (TiO₂)-.

En variante, avantageusement et selon l'invention, on choisit un catalyseur dans le groupe formé des alcoxydes métalliques. Avantageusement, on choisit un catalyseur dans le groupe formé du Ti(OR)₄ et du Zr(OR)₄ dans lesquels R est un alkyle.

En variante, avantageusement et selon l'invention, on choisit un catalyseur dans le groupe formé des acétylacétonates métalliques, par exemple l'acétylacétonate d'aluminium.

En variante, avantageusement et selon l'invention, on choisit un catalyseur dans le groupe formé des alkyles métalliques, par exemple (CH₃-CH₂)₂Zn à titre de catalyseur organo-métallique.

En variante, avantageusement et selon l'invention, on choisit un catalyseur dans le groupe formé des bases minérales, par exemple du carbonate de potassium (K₂CO₃), du carbonate de sodium (Na₂CO₃) et de l'hydroxyde de potassium (KOH), l'hydroxyde de sodium (NaOH), l'hydroxyde de barium (BaOH). Une telle base minérale utilisée à titre de catalyseur dans un procédé selon l'invention permet la formation d'un amorceur organique nucléophile -notamment d'un alcoolate- apte à réagir avec le cyclo-carbonate du précurseur et à former par décarboxylation une liaison éther. En particulier, on choisit un tel catalyseur dans le groupe formé des bases minérales aptes à augmenter la nucléophilie de l'amorceur organique, notamment pour former un hétérooligomère d'ester carbonique linéaire de glycérol α/α'-alcoxylé comprenant au moins un motif glycérol non carbonaté.

Avantageusement, on choisit le catalyseur dans le groupe formé des catalyseurs homogènes. Avantageusement, il est aussi possible de choisir le catalyseur dans le groupe formé des catalyseurs hétérogènes.

En variante, avantageusement et selon l'invention, on choisit un catalyseur organométallique.

Selon les hétéroatomes constitutifs de l'amorceur organique, on introduit dans l'oligomère des éléments hétéroatomiques -notamment de l'azote et de l'oxygène-.

Avantageusement et selon l'invention, le rapport molaire de la quantité initiale du précurseur et de la quantité initiale de l'amorceur organique dans le réacteur est compris entre 3 et 10, notamment compris entre 4 et 6. Un tel rapport molaire est adapté pour permettre un rendement de conversion du précurseur compris entre sensiblement 20% et sensiblement 80%.

L'invention concerne également les composés esters carboniques linéaires de glycérol α/α'-alcoxylés de formule (VI) générale suivante : 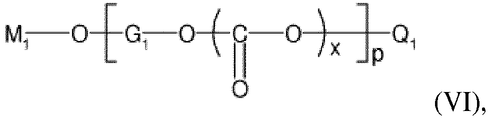 dans laquelle :
- p est un nombre entier supérieur à 1 et différent de 1 et inférieur à 500, et ;
- x est un nombre entier égal à 0 ou à 1 pouvant varier dans la formule (VI) selon chaque groupement de formule (VI-a) : 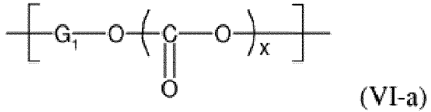 x n'étant pas toujours nul, et ;
- M₁ est choisi dans le groupe formé de l'hydrogène (H), et ;
- Q₁ représente un groupement organique comprenant au plus 10 atomes de carbone et choisi parmi les groupements hydrocarbonés aliphatiques, les groupements hydrocarbonés aminés et les groupements hydrocarbonés aminés oxygénés, et ;
- G₁ est un groupement propylique α/α'-alcoxylé de formule (II') générale suivante : 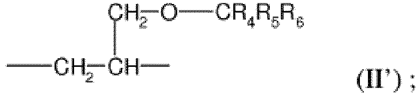 dans laquelle :
   - R₄, R₅ et R₆ sont des hydrogènes, ou
   - R₄ et R₅ sont des hydrogènes et R₆ est un groupement alkyle ou amino-alkyle présentant de 1 à 5 atomes de carbone, ou
   - R₄, R₅ et R₆ sont choisis dans le groupe formé de l'hydrogène et des groupements organiques de formule générale (VII) suivante : 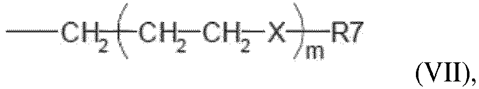 dans laquelle :
      ▪ R₇ est choisi dans le groupe formé d'un H et d'un groupement hydrocarboné présentant de 1 à 6 atomes de carbone ;
      ▪ m est un nombre entier compris entre 0 et 10 bornes incluses, et ;
      ▪ X est choisi parmi -O- et -NH-.

L'invention concerne donc des esters carboniques linéaires de glycérol α/α'-alcoxylés sous forme d'oligomères de formule générale (VI) comprenant au moins un groupement d'atomes choisi parmi les groupements propylique α/α'-alcoxylés de formule (II') et dans lesquels l'un des carbones α ou a' du glycérol du groupement propylique α/α'- alcoxylés de formule (II) est en liaison éther-oxyde covalente avec un groupement organique -notamment avec un groupement hydrocarboné-. Les composés selon l'invention sont donc des esters carboniques linéaires de glycérol α/α'-alcoxylés de formule (VI) sous forme d'oligomères. Dans ces composés, le groupement alcoxy est lié au groupement propylique α/α'-alcoxylés de formule (II') par l'atome d'oxygène de l'un des hydroxyles primaires du groupement propylique α/α'-alcoxylé de formule (II') et forme une chaine latérale dudit ester carbonique linéaire de glycérol α/α'-alcoxylé de formule (VI).

Avantageusement, la liaison éther-oxyde entre la chaine principale et chaque chaine latérale d'un ester carbonique linéaire de glycérol α/α'- alcoxylé de formule (VI) selon l'invention présente une polarité réduite par rapport à une liaison ester. Par exemple, le moment dipolaire de l'éther éthylique du carbonate de glycérol est de 4,36 Debye alors que le moment dipolaire de l'acétate de carbonate de glycérol est de 6,19 Debye.

Les esters carboniques linéaires de glycérol α/α'-alcoxylés de formule (VI) selon l'invention sont des oligomères synthétiques obtenus à partir d'une ressource naturelle renouvelable. De tels oligomères présentent au moins deux groupements α/α'-alcoxylés de formule (II) suivante : 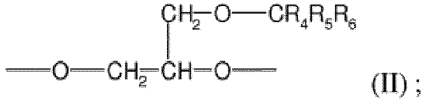 Les deux groupements α/α'-alcoxylés de formule (II) étant liés l'un à l'autre par une liaison éther (-O-) ou par une liaison di-ester carbonique (-O-CO-O-).

Ces oligomères sont formés d'une chaine principale comprenant un enchainement de groupements propyliques α/α'-alcoxylés carbonatés. Ils présentent des propriétés de viscosité, de thixotropie et de polarité qui peuvent varier et être ajustées selon les besoins et les applications.

L'invention s'étend en particulier à des oligomères selon la formule (VI) comprenant entre p=2 et p=50 groupements G₁ propyliques α/α'-alcoxylés de formule (II'). Les esters carboniques linéaires de glycérol α/α'-alcoxylés selon l'invention peuvent aussi être des polymères comprenant plus de 50 -avantageusement entre 50 et 500, de préférence entre 50 et 100- groupements G₁ propyliques α/α'-alcoxylés de formule (II').

L'invention concerne avantageusement plus particulièrement des oligomères d'esters carboniques linéaires de glycérol α/α'-alcoxylés comprenant entre 2 et 10 groupements G₁ propyliques α/α'-alcoxylés de formule (II').

Avantageusement et selon l'invention Q₁ représente le groupement -CH₂-CH₂-NH₂ dans les esters carboniques linéaires de glycérol α/α'-alcoxylés de formule (V) ou de formule (VI). Avantageusement Q₃ représente le groupement -CH₂-CH₂-NH₂ dans les esters carboniques linéaires de glycérol α/α'-alcoxylés de formule (VIII) ou de formule (IX).

Avantageusement et selon l'invention, les groupements R₄, R₅ et R₆ sont des hydrogènes (H). Chaque groupement G₁ des composés de formule (VI) est alors un groupement α/α'-méthoxylé.

Selon certains modes de réalisation particuliers et avantageux de composés selon l'invention, R₄ et R₅ sont des hydrogènes (H) et R₆ est un groupement alkyle présentant de 1 à 5 atomes de carbone.

Selon d'autres modes de réalisation particuliers et avantageux de composés selon l'invention, les groupements R₄ et R₅ sont des hydrogènes (H) et le groupement R₆ est un méthyle. Chaque groupement G₁ des composés de formule (VI) est alors un groupement α/α'-éthoxylé.

Selon d'autres modes de réalisation particuliers et avantageux de composés selon l'invention, les groupements R₄ et R₅ sont des hydrogènes (H) et le groupement R₆ est un éthyle. Chaque groupement G₁ des composés de formule (VI) est donc un groupement propylique α/α'-propoxylé.

Selon d'autres modes de réalisation particuliers et avantageux de composés selon l'invention, R₄ et R₅ sont des hydrogènes (H) et R₆ est un groupement hydrocarboné aminé, notamment un groupement hydrocarboné aminé aliphatique ou un groupement hydrocarboné aminé aromatique. En particulier, R₄ et R₅ sont des hydrogènes (H) et R₆ est un groupement amino-alkyle présentant de 1 à 5 atomes de carbone.

Selon d'autres modes de réalisation particuliers et avantageux de composés, R₄ et R₅ sont des hydrogènes (H) et R₆ est un groupement méthylamine de formule : -CH₂-NH₂.

Avantageusement et selon l'invention, R₄, R₅ et R₆ sont choisis dans le groupe formé de l'hydrogène (H) et des groupements organiques de formule générale (VII) suivante : 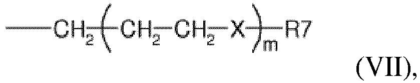 dans laquelle :
▪ R₇ est choisi dans le groupe formé d'un H et d'un groupement hydrocarboné présentant de 1 à 6 atomes de carbone ;
▪ m est un nombre entier compris entre 0 et 10 bornes incluses, et ;
▪ X est choisi parmi -O- et -NH-.

Les esters carboniques linéaires de glycérol α/α'-alcoxylés selon l'invention conformes à la formule (VI) peuvent présenter au moins une chaine latérale présentant au moins un atome ou groupement d'atomes choisi parmi un atome d'oxygène (-O-), une amine secondaire (-NH-) et une amine tertiaire.

L'invention concerne plus particulièrement les esters carboniques linéaires de glycérol α/α'-alcoxylés sous forme d'oligomères selon la formule générale (VIII) suivante : 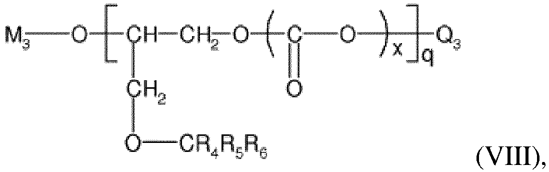 dans laquelle :
- q est un nombre entier supérieur à 1 et inférieur à 500, et ;
- x est un nombre entier égal à 0 ou à 1 pouvant varier dans la formule (VIII) selon chaque groupement (VIII-a) : 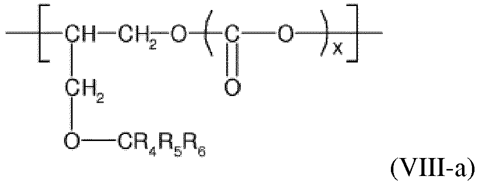 x n'étant pas toujours nul, et ;
- M₃ est l'hydrogène (H), et ;
- Q₃ représente un groupement organique comprenant au plus 10 atomes de carbone et choisi parmi les groupements hydrocarbonés aliphatiques, les groupements hydrocarbonés aminés et les groupements hydrocarbonés aminés oxygénés.

L'invention concerne plus particulièrement les esters carboniques linéaires de glycérol α/α'-alcoxylés sous forme d'oligomères selon la formule générale (IX) suivante : 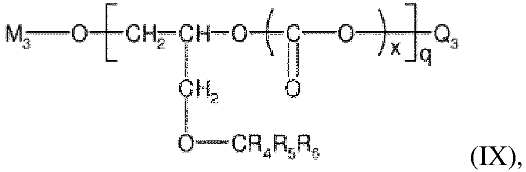 dans laquelle ;
- q est un nombre entier supérieur à 1 et inférieur à 500, et ;
- x est un nombre entier égal à 0 ou à 1 pouvant varier dans la formule (IX) selon chaque groupement de formule (IX-a) : 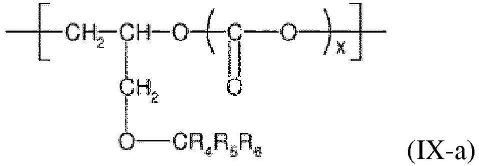 x n'étant pas toujours nul, et ;
- M₃ est choisi dans le groupe formé de l'hydrogène (H), et ;
- Q₃ représente un groupement organique comprenant au plus 10 atomes de carbone et choisi parmi les groupements hydrocarbonés aliphatiques, les groupements hydrocarbonés aminés et les groupements hydrocarbonés aminés oxygénés. En particulier, comme q est supérieur à 1, ces composés sont des oligomères.

Un oligomère ester carbonique linéaire de glycérol α/α'-alcoxylé selon l'invention comprend au moins un premier groupement α/α'-alcoxylés de formule (II) dans lequel :
- l'atome d'oxygène de l'un des carbones (a, a') du premier groupement glycérol a/a'-alcoxylé de formule (II) est engagé dans une liaison éther-oxyde avec un groupement organique de formule générale -CR₄R₅R₆, et ;
- l'atome d'oxygène de l'autre carbone (a, a') du premier groupement a/a'-alcoxylé de formule (II) et l'atome d'oxygène du carbone (β) du premier groupement α/α'-alcoxylé de formule (II) sont engagés l'un dans une liaison avec un groupement di-ester carbonique (-O-CO-O-) ou dans une liaison éther (-O-) avec un deuxième groupement α/α'-alcoxylé de formule (II), et l'autre dans une liaison éther avec un groupement d'atomes de formule M₁-O- ou M₃-O-.

Avantageusement, les esters carboniques linéaires de glycérol α/α'-alcoxylés selon l'invention présentent par analyse en spectrométrie infrarouge une bande de vibration comprise entre 1730 cm⁻¹ et 1750 cm⁻¹.

Des esters carboniques linéaires de glycérol α/α'-alcoxylés selon une première variante de l'invention sont des homooligomères d'esters carboniques de glycérol α/α'-alcoxylés de formule (V) générale suivante : 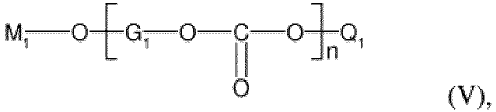 dans laquelle n est un nombre entier supérieur à 1 et différent de 1.

Un tel homooligomère d'ester carbonique glycérol α/α'-alcoxylés de formule (V) présente une répétition d'un même groupement de formule (V-a) suivante : 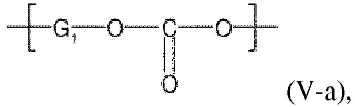 ce groupement de formule (V-a) étant présent au moins deux fois dans la chaine principale.

En particulier, l'invention concerne des homooligomères d'esters carboniques linéaires de glycérol α/α'-alcoxylés de formule (VI) dans lesquels x est constant et toujours égal à 1 dans tous les groupements de formule (VI-a).

Les oligomères esters carboniques linéaires de glycérol α/α'-alcoxylés selon l'invention peuvent être des hétérooligomères, c'est-à-dire présenter au moins un groupement carbonate propylique α/α'-alcoxylé (x = 1) et au moins un groupement choisi dans le groupe formé des groupements propyliques α/α'-alcoxylés (x = 0). Ces hétérooligomères d'esters carboniques linéaires de glycérol alcoxylés peuvent être aussi formés d'au moins un groupement carbonate propyliques α/α'-alcoxylé (x = 1) et d'au moins un groupement propyliques α/α'-alcoxylé (x = 0) enchainés les uns aux autres.

Les hétérooligomères d'esters carboniques linéaires de glycérol α/α'-alcoxylés selon l'invention peuvent présenter une alternance -régulière ou irrégulière- de motifs esters carboniques linéaires de glycérol alcoxylés (x = 1) et de motifs glycérol alcoxylé (x = 0).

M₁ et M₃ sont des hydrogènes (H). La chaine principale d'un composé selon l'invention comprend un hydroxyle unique à l'une des extrémités de ladite chaine principale.

Avantageusement, un composé selon l'invention comprend sur au moins une partie de la longueur de sa chaine principale et formant celle-ci, un homooligomère d'ester carbonique linéaire de glycérol α/α'-alcoxylé.

Dans un oligomère selon l'invention, au moins deux des trois atomes de carbone du groupement α/α'-alcoxylés de formule (II) constituent -notamment en combinaison avec les groupements esters carboniques linéaires- la chaine principale de l'oligomère.

L'invention concerne plus particulièrement les esters carboniques de glycérol α/α'-alcoxylés sous forme d'oligomères selon la formule (VI) et comprenant au moins un groupement aminé choisi dans le groupe formé des amines primaires, des amines secondaires et des amines tertiaires. De tels esters carboniques de glycérol α/α'-alcoxylés aminés selon la formule (VI) présentent des propriétés d'adhésion améliorées avec une surface métallique et sur une surface cellulosique.

Q₁ et Q₃ sont choisis indépendamment l'un de l'autre dans le groupe formé des groupements organiques choisis parmi les groupements hydrocarbonés aliphatiques, les groupements hydrocarbonés aminés et les groupements hydrocarbonés aminés oxygénés et présentant au plus 10 -notamment 5- atomes de carbone.

Avantageusement, Q₁, et Q₃ sont choisis dans le groupe formé des groupements hydrocarbonés aliphatiques.

Avantageusement, Q₁, et Q₃ sont choisis dans le groupe formé des groupements hydrocarbonés aminés. Avantageusement, Q₁, et Q₃ sont -CH₂-CH₂-NH₂.

Avantageusement, Q₁ et Q₃ sont choisis dans le groupe formé des groupements hydrocarbonés aminés oxygénés.

Avantageusement un ester carbonique linéaire de glycérol α/α'-alcoxylé de formule (VI) selon l'invention présente une masse molaire supérieure à 200 g/mole, notamment supérieure à 600 g/mole, en particulier supérieure à 1000 g/mole, préférentiellement comprise entre 1000 g/mole et 3000 g/mole, plus préférentiellement de l'ordre de 2500 g/mole.

Avantageusement un ester carbonique linéaire de glycérol α/α'-alcoxylé de formule (VI) selon l'invention présente un indice d'hydroxyle exprimé en milligramme de KOH par gramme de composé calculé selon la norme NF T600-213 compris entre 200 et 2000 mg KOH/g de composé.

L'invention concerne aussi une composition -notamment une composition liquide- comprenant au moins un oligomère ester carbonique linéaire de glycérol α/α'-alcoxylé selon l'invention.

Avantageusement et selon l'invention, la composition comprend en mélange une pluralité d'oligomères esters carboniques linéaires de glycérol α/α'-alcoxylés, chaque oligomère ester carbonique linéaire de glycérol α/α'-alcoxylé de la composition organique répondant à au moins l'une des formules générales (V), (VI), (VIII) et (IX).

Une composition selon l'invention est une composition organique qui comprend en outre avantageusement au moins un composé choisi dans le groupe formé des cyclo-carbonates de glycérol α/α'-alcoxylés de formule (XXII).

L'invention concerne également un procédé de synthèse d'esters carboniques linéaires de glycérol α/α'-alcoxylés -notamment sous forme d'oligomères-, des esters carboniques linéaires de glycérol α/α'-alcoxylés sous forme d'oligomères, une composition incorporant au moins un tel ester carbonique linéaire de glycérol α/α'-alcoxylé sous forme d'oligomère, et leurs utilisations caractérisés en combinaison par tout ou partie des caractéristiques mentionnées ci-dessus ou ci-après.

D'autres buts, caractéristiques et avantages de l'invention apparaîtront à la lecture de la description suivante comprenant les exemples donnés uniquement à titre non limitatif.

Pour la mise en œuvre d'un procédé de synthèse d'esters carboniques linéaires de glycérol α/α'-alcoxylés on met en contact une quantité d'au moins un précurseur de formule (XXII), une quantité d'au moins un catalyseur et une quantité d'au moins un amorceur organique. Il est possible d'obtenir le précurseur cyclo-carbonate de glycérol α/α'-alcoxylé de formule (XXII) par un procédé dérivé du procédé décrit dans FR 2 778 182.

Ainsi, pour mettre en œuvre un procédé de synthèse selon l'invention, on réalise d'abord une O-éthérification du carbonate de glycérol de façon à former le précurseur cyclo-carbonate de glycérol α/α'-alcoxylés de formule (XXII) puis on soumet le précurseur à un procédé de synthèse selon l'invention et de façon à former un oligomère ester carbonique linéaire de glycérol α/α'-alcoxylé de formule générale (VI) dans lequel le groupement alcoxyle est lié à l'oligomère par un groupement méthylène espaceur de la chaine principale de l'oligomère.

Il est aussi possible d'obtenir le précurseur cyclo-carbonate de glycérol α/α'-alcoxylé de formule (XXII) par d'autres voies de synthèse, notamment par réaction :
- d'un éther de glycérol de formule (XXVI) : 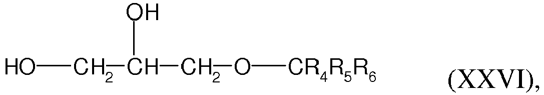 avec ;
- un carbonate choisi dans le groupe formé des carbonates de dialkyle -notamment du carbonate de diméthyle et du carbonate de diéthyle-, et des carbonates d'alkylène -notamment du carbonate d'éthylène et du carbonate de propylène- ;
en présence de carbonate de potassium (K₂CO₃) à une température de l'ordre de 75°C. Des variantes d'une telle voie de synthèse sont décrites aux exemples 1 et 2.

Il est aussi possible d'obtenir le précurseur cyclo-carbonate de glycérol alcoxylé de formule (XXII) par réaction :
- du glycérol, avec ;
- un carbonate choisi dans le groupe formé des carbonates de dialkyle -notamment du carbonate de diméthyle et du carbonate de diéthyle-, et des carbonates d'alkylène -notamment du carbonate d'éthylène et du carbonate de propylène- ;
à une température supérieure à 200°C -notamment de l'ordre de 230°C- en présence d'alumine (Al₂O₃). Une variante d'une telle voie de synthèse est décrite à l'exemple 3.

Il est aussi possible de préparer un cyclo-carbonate de glycérol α/α'-alcoxylé de formule (XXII) par réaction :
- du cyclo-carbonate de glycérol, et ;
- un carbonate choisi dans le groupe formé des carbonates de dialkyle -notamment du carbonate de diméthyle et du carbonate de diéthyle-, et des carbonates d'alkylène -notamment du carbonate d'éthylène et du carbonate de propylène- ;
à une température de l'ordre de 200°C en présence d'alumine (Al₂O₃). Une variante d'une telle voie de synthèse est décrite à l'exemple 4.

Il est aussi possible de préparer un cyclo-carbonate de glycérol α/α'-alcoxylé de formule (XXII) par réaction de trans-carbonatation catalytique du glycérol à partir d'un carbonate organique cyclique.

Il est aussi possible de préparer un cyclo-carbonate de glycérol α/α'-alcoxylé de formule (XXII) par réaction d'un alcool -notamment de méthanol, d'éthanol, d'*iso*-propanol ou de *tert*-butanol- sur le glycérol en présence d'une résine cationique (par exemple, une résine Amberlyst®, une zéolithe, un acide sulfonique greffé sur silice, une silice mésoporeuse) à titre de catalyseur et à une température comprise entre 60°C et 200°C.

### EXEMPLE 1 - Carbonatation de l'α-méthyl-éther de glycérol.

Dans un ballon en verre de 100 ml on place 1 équivalent molaire d'a-méthyl-éther de glycérol (3-méthoxy-1,2-propanediol, CAS 623-39-2) et 3 équivalents molaires de carbonate de diméthyle (CAS 616-38-6) sous agitation mécanique rotative à une vitesse de 400 rotations par minute (rpm) et à la température de 75°C en présence de 0,03 équivalents molaires de carbonate de potassium (CAS 584-08-7) à titre de catalyseur pendant 8 heures. On forme l'éther méthylique du cyclo-carbonate de glycérol (CGME) avec un rendement après isolement de 85% par rapport à l'α-méthyl-éther de glycérol de départ.

### EXEMPLE 2 - Carbonatation de l'α-éthyl-éther de glycérol.

Dans un ballon en verre de 100 mL on place 1 équivalent molaire l'α,-éthyl-éther de glycérol (3-éthoxy-1,2-propanediol, CAS 1874-62-0) et 3 équivalents molaires de carbonate de diméthyle (CAS 616-38-6) sous agitation mécanique rotative à une vitesse de 400 rotations par minute (rpm) et à la température de 75°C en présence de 0,03 équivalents molaires de carbonate de potassium (CAS 584-08-7) à titre de catalyseur pendant 8 heures. On forme l'éther éthylique du cyclo-carbonate de glycérol avec un rendement de 85% après isolement.

### EXEMPLE 3 - α-méthylation et carbonatation du glycérol.

On prépare l'éther méthylique du cyclo-carbonate de glycérol (CGME) à partir du glycérol. Dans un autoclave on place 1 équivalent molaire de glycérol (CAS 56-81-5) et 10 équivalents molaires de carbonate de diméthyle (CAS 616-38-6) à la température de 230°C pendant 3 heures en présence de 2 équivalents molaires d'alumine (Al₂O₃) neutre et sous agitation mécanique rotative à 400 rpm. On obtient l'éther méthylique du cyclo-carbonate de glycérol (CGME) avec un rendement de 50% déterminé par chromatographie en phase gazeuse couplée à un détecteur à ionisation de flamme (GC-FID). On isole le CGME par distillation sous pression réduite (18 hPa) et à la température de 115°C.

### EXEMPLE 4 - α-méthylation du cyclo-carbonate de glycérol.

On prépare l'éther méthylique du cyclo-carbonate de glycérol (CGME) à partir du cyclo-carbonate de glycérol. Dans un autoclave on place 1 équivalent molaire de cyclo-carbonate de glycérol (CAS 931-40-8, 4-hydroxymethyl-1,3-dioxolan-2-one ou 4-hydroxymethyl-2-oxo-1,3-dioxolane) et 5 équivalents molaires de carbonate de diméthyle (CAS 616-38-6) à la température de 200°C pendant 4 heures en présence de 1 équivalent molaire d'alumine (Al₂O₃) neutre et sous agitation mécanique rotative à 400 rpm. On obtient l'éther méthylique du cyclo-carbonate de glycérol (CGME) avec un rendement de 66% déterminé par analyse du milieu réactionnel par chromatographie en phase gazeuse couplée à un détecteur à ionisation de flamme (GC-FID). On isole le CGME par distillation sous pression réduite (18 hPa) et à la température de 115°C.

A titre indicatif, il est aussi possible de préparer le cyclo-carbonate de glycérol selon un procédé décrit dans le document FR 2 733 232 par trans-carbonatation catalytique du glycérol à partir d'un carbonate organique cyclique.

### EXEMPLE 5 - Oligomérisation de l'éther méthylique de cyclo-carbonate de glycérol (CGME) en présence de glycérol à titre d'amorceur organique d'oligomérisation.

Dans un autoclave de 250 mL on place une quantité de CGME, une quantité de glycérol à titre d'amorceur d'oligomérisation et une quantité de sulfate de zinc à titre de catalyseur. Avant la réaction, la proportion massique de CGME et de glycérol est de 85/15 et la proportion massique de catalyseur est de 0,5% par rapport à la masse totale des réactifs. Après la fermeture hermétique aux gaz de l'autoclave, on chauffe l'autoclave de façon à porter la température du milieu réactionnel à 180°C. A cette température de 180°C, on met le milieu réactionnel à pression atmosphérique et on maintient cette température de réaction pendant une durée de 2 heures sous agitation mécanique rotative à 400 rpm.

Par analyse en spectroscopie infrarouge à transformée de Fourrier (FTIR) du mélange réactionnel, on observe une forte diminution de l'intensité de la bande de vibration correspondant à la fréquence de vibration du carbonyle du CGME à 1790 cm⁻¹, et l'apparition d'une bande correspondant à la fréquence de vibration du carbonyle du carbonate linéaire à 1750-1730 cm⁻¹.

### EXEMPLE 6 - Oligomérisation de l'éther méthylique de cyclo-carbonate de glycérol (CGME) en présence de glycérol à titre d'amorceur d'oligomérisation.

Dans un autoclave de 250 mL on place une quantité de CGME, une quantité de glycérol à titre d'amorceur d'oligomérisation et une quantité de stéarate de zinc (CAS 557-05-1, bis-octadécanoate de zinc) à titre de catalyseur. Avant la réaction, la proportion massique de CGME et de glycérol est de 85/15 et la proportion massique de catalyseur est de 0,5% par rapport à la masse totale des réactifs. Après la fermeture hermétique aux gaz de l'autoclave, on chauffe l'autoclave de façon à porter la température du milieu réactionnel à 180°C. A cette température de 180°C, on met le milieu réactionnel à pression atmosphérique et on maintient cette température de réaction pendant une durée de 2 heures sous agitation mécanique rotative à 400 rpm.

Par analyse RMN ¹H, on observe à 4,3 ppm et 3,57 ppm, les signaux correspondant aux protons du glycérol en positions 1-3 et 1-4 par rapport au carbone du carbonate linéaire. On observe aussi entre 3,2 ppm et 3,5 ppm, les signaux correspondant aux protons du glycérol en positions 1-2 et 1-3 par rapport au groupement éther-oxyde de glycérol.

Le taux de conversion du CGME (disparition du groupement cyclo-carbonate), le taux de conversion de l'amorceur d'oligomérisation (glycérol) et la masse molaire moyenne de l'oligomère formé sont donnés au tableau 1 ci-après.

**Tableau 1**

| Amorceur d'oligomérisation | Taux de conversion du CGME, % | Taux de conversion de l'amorceur, % | Masse molaire moyenne de l'oligomère, g/mole |
|---|---|---|---|
| Glycérol | 95,3 | 88,7 | 2410 |

### EXEMPLE 7 - Oligomérisation de l'éther méthylique de cyclo-carbonate de glycérol (CGME) en présence d'éthanolamine à titre d'amorceur d'oligomérisation.

Dans un autoclave de 250 mL on place une quantité de CGME, une quantité d'éthanolamine (CAS 141-43-5, 2-amino-1-éthanol) à titre d'amorceur d'oligomérisation et une quantité de stéarate de zinc (CAS 557-05-1, bis-octadécanoate de zinc). Avant la réaction, la proportion massique de CGME et de glycérol est de 85/15 et la proportion de catalyseur est de 0,5% par rapport à la masse totale des réactifs. Après la fermeture hermétique aux gaz de l'autoclave, on chauffe l'autoclave de façon à porter la température du milieu réaction à 180°C. A cette température de 180°C, on met le milieu réactionnel à pression atmosphérique et on maintien cette température de réaction pendant une durée de 2 heures sous agitation mécanique rotative à 400 rpm.

Le taux de conversion du CGME (disparition du groupement cyclo-carbonate), le taux de conversion de l'amorceur d'oligomérisation (2-amino-1-éthanol) et la masse molaire moyenne des oligomères esters carboniques de glycérol α/α'-alcoxylés formés sont donnés au tableau 2 ci-après.

**Tableau 2**

| Amorceur d'oligomérisation | Taux de conversion du CGME, % | Masse molaire moyenne de l'oligomère, g/mole |
|---|---|---|
| 2-amino-1-éthanol | 100 | 2460 |
| 2-amino-1-éthanol | 100 | 2440 |

On détermine que 16% à 20% des groupes aminés de l'éthanolamine de départ sont présents dans l'oligomère obtenu.

L'analyse par spectroscopie infra-rouge par transformée de Fourrier de la composition obtenue par traitement du CGME par l'éthanolamine (EA) montre la disparition complète du signal à 1794 cm⁻¹ correspondant au carbonyle du précurseur CGME et l'apparition d'un signal à 1750 cm⁻¹ correspondant au carbonyle de l'ester carbonique linéaire de l'oligomère obtenu. Cette analyse ne montre aucun signal à 1710 cm⁻¹ correspondant au carbonyle d'une fonction uréthane linéaire.

### EXEMPLE 8 - Oligomérisation d'un mélange de CGME et de cyclo-carbonate de glycérol en présence d'éthanolamine à titre d'amorceur d'oligomérisation.

Dans un autoclave de 250 mL on place une quantité de CGME, une quantité de cyclo-carbonate de glycérol, une quantité d'éthanolamine (CAS 141-43-5, 2-amino-1-éthanol) à titre d'amorceur d'oligomérisation et une quantité de stéarate de zinc (CAS 557-05-1, octadécanoate de zinc). Avant la réaction, la proportion massique de CGME, de cyclo-carbonate de glycérol et d'éthanolamine est de 42,5/42,5/15 et la proportion de catalyseur est de 0,5% par rapport à la masse totale des réactifs. Après la fermeture hermétique aux gaz de l'autoclave, on chauffe l'autoclave de façon à porter la température du milieu réaction à 180°C. A cette température de 180°C, on met le milieu réactionnel à pression atmosphérique et on maintient cette température de réaction pendant une durée de 2 heures sous agitation mécanique rotative à 400 rpm.

### EXEMPLE 9 - Oligomérisation de l'éther méthylique de cyclo-carbonate de glycérol (CGME) en présence de 3-(2-hydroxyéthyl)imidazolidin-2-one (HEI) à titre d'amorceur d'oligomérisation.

Dans un autoclave de 250 mL on place du CGME, du HEI à titre d'amorceur d'oligomérisation et du stéarate de zinc (CAS 557-05-1, octadécanoate de zinc). Avant la réaction, la proportion massique de CGME et de glycérol est de 85/15 et la proportion de catalyseur est de 0,5% par rapport à la masse totale des réactifs. Après la fermeture hermétique aux gaz de l'autoclave, on chauffe l'autoclave de façon à porter la température du milieu réaction à 180°C. A cette température de 180°C, on met le milieu réactionnel à pression atmosphérique et on maintient cette température de réaction pendant une durée de 2 heures sous agitation mécanique rotative à 400 rpm.

Le taux de conversion du CGME (disparition du groupement cyclo-carbonate), le taux de conversion de l'amorceur d'oligomérisation (HEI) et la masse molaire moyenne de l'oligomère formé sont donnés au tableau 3 ci-après.

**Tableau 3**

| Amorceur d'oligomérisation | Taux de conversion du CGME, % | Taux de conversion de l'amorceur, % | Masse molaire moyenne de l'oligomère, g/mole |
|---|---|---|---|
| HEI | 75,5 | 91,1 | 2460 |

### EXEMPLE 10 -Oligomérisation de l'éther méthylique de cyclo-carbonate de glycérol (CGME) en présence de glycérol.

Dans un autoclave de 250 mL on place à température ambiante une quantité de CGME, une quantité de glycérol et une quantité d'un catalyseur.

On ferme hermétiquement l'autoclave, puis on chauffe la composition formée de façon à porter sa température à la température de réaction. Lorsque cette température de réaction est atteinte, on met le réacteur à pression atmosphérique et on maintient la composition formée à cette température de réaction sous agitation mécanique rotative à 400 rpm.

La température de réaction, la nature du catalyseur, la durée de réaction, le rapport massique du précurseur (CGME) et du glycérol et le taux de conversion du précurseur sont donnés au tableau 4 ci-après.

**Tableau 4**

| Température de réaction, °C | Catalyseur | Durée de réaction, h | CGME/Glycérol, m/m | Conversion, % |
|---|---|---|---|---|
| 180 | Stéarate de Zn | 2 | 20 | 10 |
| 180 | Stéarate de Zn | 2 | 10 | 19 |
| 180 | Stéarate de Zn | 3 | 5 | 72 |
| 180 | Stéarate de Zn | 6 | 5 | 48,5 |
| 180 | Stéarate de Zn | 3 | 4,5 | 77 |
| 160 | Stéarate de Zn | 2 | 4,5 | 50 |
| 200 | Stéarate de Zn | 2 | 4,5 | 64 |
| 180 | ZnSO₄ | 2 | 5,0 | 15,5 |
| 180 | K₂CO₃ | 2 | 5,9 | 100 |

### EXEMPLE 11 - Oligomérisation de l'éther méthylique de cyclo-carbonate de glycérol (CGME) catalysée par le stéarate de zinc en présence d'un amorceur d'oligomérisation.

Dans un autoclave de 250 mL on place à température ambiante une quantité de CGME, une quantité d'un amorceur d'oligomérisation choisi parmi la triéthanolamine (TEA), l'éthanolamine (EA), l'éthane-1,2-diamine (EDA), le carbonate de glycérol pentacyclique (CG), l'α-éther méthylique de glycérol (EG), l'éthanol (EtOH) et le n-butanol (BuOH) et une quantité de stéarate de zinc.

On ferme hermétiquement l'autoclave, puis on chauffe la composition formée de façon à porter sa température à la température de réaction. Lorsque cette température de réaction est atteinte, on met le réacteur à pression atmosphérique et on maintient la composition formée à cette température de réaction sous agitation mécanique rotative à 400 rpm pendant 2 heures.

La température de réaction, la nature chimique de l'amorceur d'oligomérisation, le rapport massique du précurseur (CGME) et de l'amorceur et le taux de conversion du précurseur sont donnés au tableau 5 ci-après.

**Tableau 5**

| Température, °C | Amorceur | CGME/amorceur, m/m | Conversion, % |
|---|---|---|---|
| 180 | TEA | 7,5 | 89,0 |
| 160 | EA | 3 | 84,4 |
| 180 | EA | 3 | 98,8 |
| 180 | EDA | 3,2 | 97,0 |
| 180 | CG | 5,5 | 19,8 |
| 180 | EG | 4,4 | 19,5 |
| 180 | EtOH | 2,2 | 11,4 |
| 180 | BuOH | 3,5 | 19,2 |

L'analyse par spectroscopie infra-rouge par transformée de Fourrier de la composition obtenue par traitement du CGME par l'éthanolamine (EA) montre la disparition complète du signal à 1794 cm⁻¹ correspondant au carbonyle du précurseur CGME et l'apparition d'un signal à 1750 cm⁻¹ correspondant au carbonyle de l'ester carbonique linéaire de l'oligomère obtenu.

L'analyse par spectroscopie infra-rouge par transformée de Fourrier de la composition obtenue par traitement du CGME par l'éthane-1,2-diamine (EDA) montre la disparition complète du signal à 1794 cm⁻¹ correspondant au carbonyle du précurseur CGME et l'apparition d'un signal majoritaire à 1710 cm⁻¹ correspondant au carbonyle d'un groupement uréthane et d'un signal minoritaire à 1750 cm⁻¹ correspondant au carbonyle du carbonate linéaire.

**Tableau 6**

| Amorceur / Température °C | Mn | Mw | Mz | Ip |
|---|---|---|---|---|
| EA / 160 | 740 | 781 | 826 | 1,06 |
| | 290 | 294 | 299 | 1,02 |
| | 183 | 185 | 186 | 1,01 |
| | 104 | 105 | 107 | 1,02 |
| EA / 180 | 736 | 760 | 787 | 1,03 |
| | 418 | 422 | 427 | 1,01 |
| | 288 | 290 | 293 | 1,01 |
| | 177 | 180 | 183 | 1,02 |
| TEA / 180 | 421 | 428 | 438 | 1,02 |
| | 292 | 295 | 298 | 1,01 |
| | 170 | 173 | 175 | 1,01 |
| Glycérol / 180 | 409 | 411 | 413 | 1,00 |
| | 298 | 302 | 305 | 1,01 |
| | 193 | 195 | 197 | 1,01 |
| Glycérol / K₂CO₃ | 637 | 705 | 821 | 1,11 |
| | 395 | 398 | 401 | 1,01 |
| | 291 | 294 | 296 | 1,01 |
| Glycérol / ZnSO₄ | 177 | 180 | 183 | 1,02 |

L'analyse par chromatographie en perméation de gel (GPC) des compositions obtenues aux exemples 9 et 10 et décrites aux tableaux 4 et 5 est présentée au tableau 6 ci-dessus dans lequel :
- Mn est la masse moléculaire en nombre de chaque oligomère de la composition exprimée en g/mole ;
- Mw est la masse moléculaire en poids de chaque oligomère de la composition exprimée en g/mole ;
- Mz est la moyenne de centrifugation de chaque oligomère de la composition exprimée en g/mole ;
- Ip est l'indice de polydispersité de chaque oligomère de la composition.

### EXEMPLE 12 - Oligomérisation de l'éther méthylique de cyclo-carbonate de glycérol (CGME) catalysée par le stéarate de zinc en présence d'un amorceur organique d'oligomérisation.

Dans un autoclave de 250 mL on place à température ambiante environ 30 g de CGME, de l'éthanolamine (EA) à titre d'amorceur d'oligomérisation et une quantité de stéarate de zinc.

On ferme hermétiquement l'autoclave, puis on chauffe la composition formée de façon à porter sa température à la température de réaction. Lorsque cette température de réaction est atteinte, on met le réacteur à pression atmosphérique et on maintient la composition formée à cette température de réaction sous agitation mécanique rotative à 400 rpm.

La température de réaction, le rapport molaire du CGME et de l'éthanolamine et la masse moléculaire en nombre Mnₓ de chaque oligomère de la composition obtenue (exprimée en g/mole) sont donnés au tableau 7 ci-après.

**Tableau 7**

| Température, °C | CGME/EA, m/m | Mn₁ | Mn₂ | Mn₃ | Mn₄ |
|---|---|---|---|---|---|
| 180 | 10 | 691 | 411 | 290 | 179 |
| 200 | 3 | 682 | 412 | 288 | 180 |
| 180 | 3 | 736 | 418 | 288 | 177 |
| 160 | 3 | 740 | 290 | 183 | 104 |

Bien entendu, cette description est donnée à titre d'exemple illustratif uniquement et l'homme du métier pourra y apporter de nombreuses modifications, variantes et applications sans sortir de la portée de l'invention, comme par exemple le choix de l'amorceur organique d'oligomérisation, le choix du précurseur cyclo-carbonate de glycérol alcoxylé et le choix du catalyseur.

## Revendications

1. Procédé de synthèse d'esters carboniques linéaires de glycérol α/α'-alcoxylés dans lequel on met en contact :
i. une quantité d'au moins un précurseur choisi dans le groupe formé des cyclo-carbonates de glycérol a/a'-alcoxylés de formule (XXII) suivante : 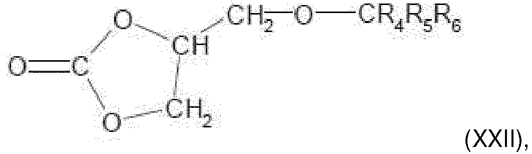 dans laquelle :
- R₄, R₅ et R₆ sont des hydrogènes, ou
- R₄ et R₅ sont des hydrogènes et R₆ est un groupement alkyle ou amino-alkyle présentant de 1 à 5 atomes de carbone, ou
- R₄, R₅ et R₆ sont choisis dans le groupe formé de l'hydrogène et des groupements organiques de formule générale (VII) suivante : 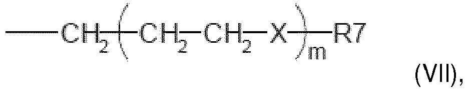 dans laquelle :
▪ R₇ est choisi dans le groupe formé d'un H et d'un groupement hydrocarboné présentant de 1 à 6 atomes de carbone ;
▪ m est un nombre entier compris entre 0 et 10 bornes incluses, et ;
▪ X est choisi parmi -O- et -NH-, et ;
ii. une quantité d'au moins un catalyseur choisi dans le groupe formé des oxydes métalliques, des alcoxydes métalliques, des acides de Lewis, des catalyseurs organo-métalliques et des bases minérales ;
iii. une quantité d'au moins un amorceur organique choisi dans le groupe formé des alcools, des polyols et des amino-alcools ;
à une température de réaction inférieure à 220°C,
**caractérisé en ce que** dans une première étape, on mélange et on chauffe le(s) précurseur(s), le(s) catalyseur(s) et l'(les) amorceur(s) organique(s) dans un réacteur clos étanche aux gaz de façon à atteindre la température de réaction et une pression, dite pression autogène, supérieure à la pression atmosphérique dans le réacteur clos, puis, à la température de réaction, dans une deuxième étape, on réalise une fuite de composition gazeuse de façon à diminuer la pression dans le réacteur et on maintient la température de réaction dans le réacteur pendant une durée supérieure à 5 min.

2. Procédé selon la revendication 1, **caractérisé en ce qu'**au moins un amorceur organique est choisi dans le groupe formé des amino-alcools présentant au moins une amine choisie dans le groupe formé des amines primaires, des amines secondaires et des amines tertiaires.

3. Procédé selon l'une des revendications 1 ou 2, **caractérisé en ce qu'**au moins un amorceur organique est la triéthanolamine.

4. Procédé selon l'une des revendications 1 à 3, **caractérisé en ce qu'**au moins un amorceur organique est choisi dans le groupe formé des amino-alcools présentant une chaine principale comprenant de 2 à 10 atomes de carbone.

5. Procédé selon l'une des revendications 1 à 4, **caractérisé en ce qu'**au moins un amorceur organique est choisi dans le groupe formé du 1-amino-1-cyclopentaneméthanol, du 4-aminocyclohexanol, de l'amino-1-butanol, du 2-amino-1-butanol, du 2-amino-1-phényl-1,3-propanediol, du 3-amino-3-phénylpropan-1-ol et du 2-amino-3-phényl-1-propan-1-ol.

6. Procédé selon l'une des revendications 1 à 5, **caractérisé en ce qu'**au moins un catalyseur est choisi dans le groupe formé du sulfate de zinc et du stéarate de zinc.

7. Composés esters carboniques linéaires de glycérol a/a'-alcoxylés de formule (VI) générale suivante : 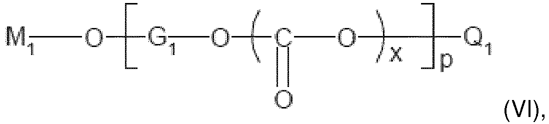 dans laquelle :
- p est un nombre entier supérieur à 1, différent de 1 et inférieur à 500 ;
- x est un nombre entier égal à 0 ou à 1 pouvant varier dans la formule (VI) selon chaque groupement de formule (VI-a) : 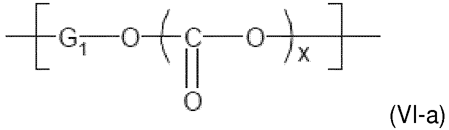 x n'étant pas toujours nul, et ;
- M₁ est l'hydrogène, et ;
- Q₁ représente un groupement organique comprenant au plus 10 atomes de carbone et choisi parmi les groupements hydrocarbonés aliphatiques, les groupements hydrocarbonés aminés et les groupements hydrocarbonés aminés oxygénés, et ;
- G₁ est un groupement propylique a/a'-alcoxylé de formule (II') générale suivante : 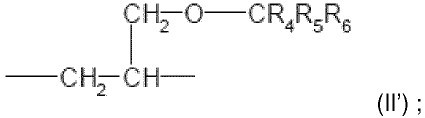 dans laquelle :
- R₄, R₅ et R₆ sont des hydrogènes, ou
- R₄ et R₅ sont des hydrogènes et R₆ est un groupement alkyle ou amino-alkyle présentant de 1 à 5 atomes de carbone, ou
- R₄, R₅ et R₆ sont choisis dans le groupe formé de l'hydrogène et des groupements organiques de formule générale (VII) suivante : 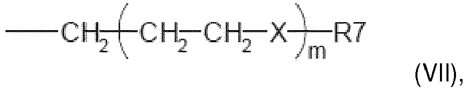 dans laquelle :
▪ R₇ est choisi dans le groupe formé d'un H et d'un groupement hydrocarboné présentant de 1 à 6 atomes de carbone ;
▪ m est un nombre entier compris entre 0 et 10 bornes incluses, et ;
▪ X est choisi parmi -O- et -NH-.

8. Composés selon la revendication 7, de formule (VIII) suivante : 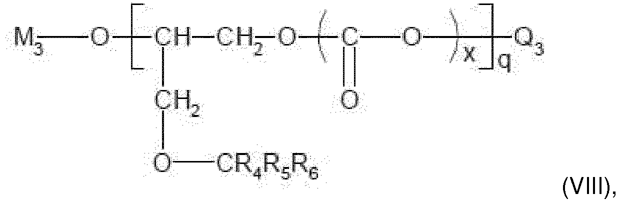 dans laquelle :
- q est un nombre entier supérieur à 1 et inférieur à 500, et ;
- x est un nombre entier égal à 0 ou à 1 pouvant varier dans la formule (VIII) selon chaque groupement (VIII-a) : 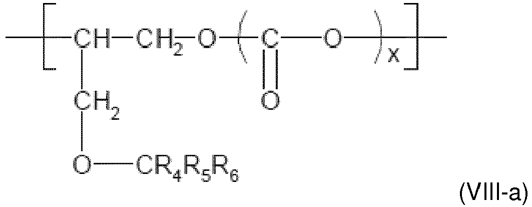 x n'étant pas toujours nul, et ;
- M₃ est l'hydrogène (H), et ;
- Q₃ représente un groupement organique comprenant au plus 10 atomes de carbone et choisi parmi les groupements hydrocarbonés aliphatiques, les groupements hydrocarbonés aminés et les groupements hydrocarbonés aminés oxygénés.

9. Composés selon la revendication 7, de formule (IX) suivante : 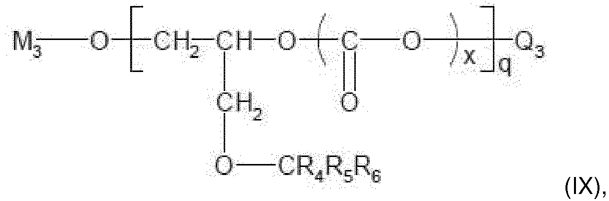 dans laquelle ;
- q est un nombre entier supérieur à 1 et inférieur à 500, et ;
- x est un nombre entier égal à 0 ou à 1 pouvant varier dans la formule (IX) selon chaque groupement de formule (IX-a) : 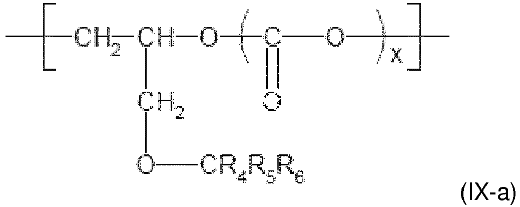 x n'étant pas toujours nul, et ;
- M₃ est l'hydrogène (H), et ;
- Q₃ représente un groupement organique comprenant au plus 10 atomes de carbone et choisi parmi les groupements hydrocarbonés aliphatiques, les groupements hydrocarbonés aminés et les groupements hydrocarbonés aminés oxygénés.

10. Composés selon la revendication 7, **caractérisé en ce que** Q₁ représente le groupement -CH₂-CH₂-NH₂.

11. Composés selon l'une des revendications 8 à 9, **caractérisé en ce que** Q₃ représente le groupement -CH₂-CH₂-NH₂.

12. Composés selon l'une des revendications 7 ou 11, de formule (V) générale suivante : 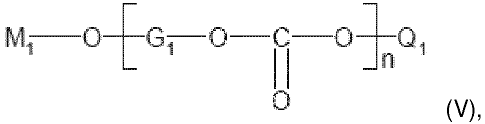 dans laquelle n est un nombre entier supérieur à 1 et différent de 1.

13. Composés selon l'une des revendications 7 à 12, **caractérisés en ce que** R₄, R₅ et R₆ sont choisis dans le groupe formé de l'hydrogène (H) et des groupements organiques de formule (VII) telle que définie à la revendication 7.

14. Composition comprenant au moins un ester carbonique linéaire de glycérol a/a'-alcoxylé selon l'une des revendications 7 à 13.

## Patentansprüche

1. Verfahren zur Synthese von linearen Carbonestern des α/α'-alkoxylierten Glyzerins, bei dem in Kontakt gebracht wird:
i. eine Menge an mindestens einer Vorstufe, die ausgewählt ist aus der Gruppe, die aus Cyclocarbonaten des α/α'-alkoxylierten Glyzerins der folgenden Formel (XXII) gebildet ist: 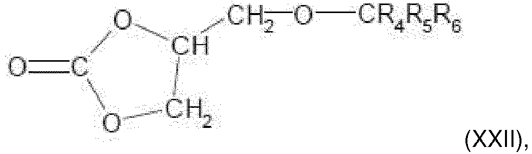 in der:
- R₄, R₅ und R₆ Wasserstoffe sind oder
- R₄ und R₅ Wasserstoffe sind und R₆ eine Alkyl- oder Aminoalkylgruppe ist, die 1 bis 5 Kohlenstoffatome aufweist oder
- R₄, R₅ und R₆ ausgewählt sind aus der Gruppe, die von Wasserstoff und organischen Gruppen der folgenden allgemeinen Formel (VII) gebildet ist: 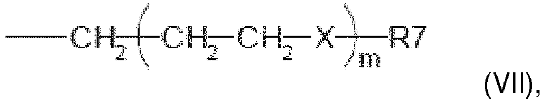 in der:
▪ R₇ ausgewählt ist aus der Gruppe, die von einem H und einer Kohlenwasserstoffgruppe, die 1 bis 6 Kohlenstoffatome aufweist, gebildet ist;
▪ m eine ganze Zahl zwischen 1 und 10, Grenzen eingeschlossen, ist und;
▪ X ausgewählt ist aus -O- und -NH-, und;
ii. eine Menge mindestens eines Katalysators ausgewählt aus der Gruppe, die von Metalloxiden, Metallalkoxiden, Lewis-Säuren, organometallischen Säuren und mineralischen Basen gebildet ist;
iii. eine Menge mindestens eines organischen Initiators ausgewählt aus der Gruppe, die von Alkoholen, Polyolen und Aminoalkoholen gebildet ist;
bei einer Reaktionstemperatur kleiner als 220 °C,
**dadurch gekennzeichnet, dass** in einem ersten Schritt der oder die Vorstufe(n), der oder die Katalysator(en) und der oder die organischen Initiator(en) derart in einem geschlossenen, gegen Gas abgedichteten Reaktor gemischt und aufgeheizt werden, dass eine Reaktionstemperatur und ein Druck, genannt autogener Druck, größer als der atmosphärische Druck in dem geschlossenen Reaktor erreicht wird, dann, bei der Reaktionstemperatur, in einem zweiten Schritt ein Austritt einer Gaszusammensetzung realisiert wird, um den Druck in dem Reaktor zu verringern und die Reaktionstemperatur in dem Reaktor während einer Dauer größer als 5 Minuten gehalten wird.

2. Verfahren nach Anspruch 1, **dadurch gekennzeichnet, dass** mindestens ein organischer Initiator ausgewählt ist aus der Gruppe, die aus den Aminoalkoholen gebildet ist, die mindestens ein Amin aufweisen, das ausgewählt ist aus der Gruppe, die von primären Aminen, sekundären Aminen und tertiären Aminen gebildet ist.

3. Verfahren nach einem der Ansprüche 1 oder 2, **dadurch gekennzeichnet, dass** mindestens ein organischer Initiator Triethanolamin ist.

4. Verfahren nach einem der Ansprüche 1 bis 3, **dadurch gekennzeichnet, dass** mindestens ein organischer Initiator ausgewählt ist aus der Gruppe, die von den Aminoalkoholen gebildet ist, die eine 2 bis 10 Kohlenstoffatome enthaltende Hauptkette aufweisen.

5. Verfahren nach einem der Ansprüche 1 bis 4, **dadurch gekennzeichnet, dass** mindestens ein organischer Initiator ausgewählt ist aus der Gruppe, die von 1-Amino-1-cyklopentanmethanol, 4-Aminocyklhexanol, Amino-1-butanol, 2-Amino-1-butanol, 2-Amino-1-phenyl-1,3-propandiol, 3-Amino-3-phenylpropan-1-ol und 2-Amino-3-phenyl-1-propan-1-ol gebildet ist.

6. Verfahren nach einem der Ansprüche 1 bis 5, **dadurch gekennzeichnet, dass** mindestens ein Katalysator ausgewählt ist aus der Gruppe, die aus Zinksulfat und Zinkstearat gebildet ist.

7. Verbindungen linearer Carbonester des α/α'-alkoxylierten Glyzerins der folgenden allgemeinen Formel (VI): 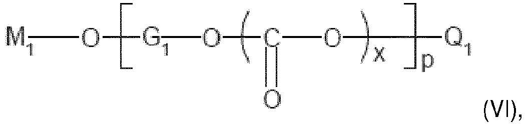 in der:
- p eine ganze Zahl größer als 1, unterschiedlich zu 1 und kleiner als 500 ist
- x eine ganze Zahl gleich 0 oder 1 ist, die in der Formel (VI) gemäß jeder Gruppe der Formel (VI-a) variieren kann: 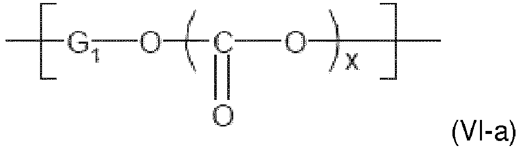 wobei x nicht immer null ist, und;
- M₁ Wasserstoff ist und;
- Q₁ eine organische Gruppe darstellt, die höchstens 10 Kohlenstoffatome enthält und ausgewählt ist aus den aliphatischen Kohlenwasserstoffgruppen, den Amino-Kohlenwasserstoffgruppen und den oxygenierten Amino-Kohlenwasserstoffgruppen, und;
- G₁ eine α/α'-alkoxylierte Propylgruppe der folgenden allgemeinen Formel (II') ist: 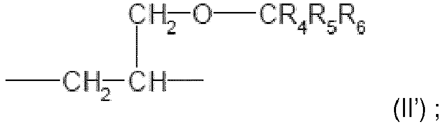 in der:
- R₄, R₅, und R₆ Wasserstoffe sind, oder
- R₄ und R₅ Wasserstoffe sind und R₆ eine Alkyl- oder Aminoalkylgruppe ist, die 1 bis 5 Kohlenstoffatome aufweist, oder
- R₄, R₅ und R₆ ausgewählt sind aus der Gruppe, die von Wasserstoff und organischen Gruppen der folgenden allgemeinen Formel (VII) gebildet ist: 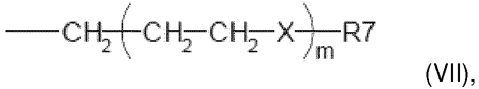 in der:
▪ R₇ ausgewählt ist aus der Gruppe, die von einem H und einer Kohlenwasserstoffgruppe, die1 bis 6 Kohlenstoffatomen aufweist, gebildet ist;
▪ m eine ganze Zahl zwischen 1 und 10, Grenzen eingeschlossen, ist und;
▪ X ausgewählt ist aus -O- und -NH-.

8. Verbindungen nach Anspruch 7 der folgenden Formel (VIII): 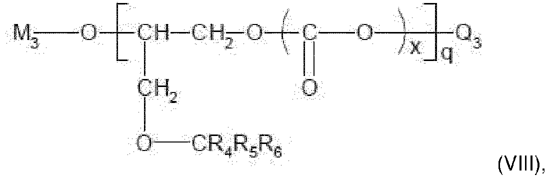 in der:
- q eine ganze Zahl größer als 1 und kleiner als 500 ist und;
- x eine ganze Zahl gleich 0 oder 1 ist, die in der Formel (VIII) gemäß jeder Gruppe der Formel (VIII-a) variieren kann: 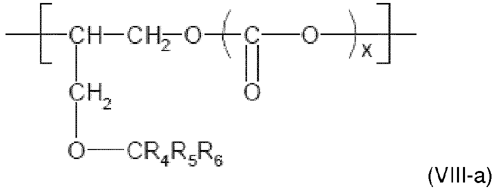 wobei x nicht immer null ist, und;
- M₃ Wasserstoff (H) ist und;
- Q₃ eine organische Gruppe darstellt, die höchstens 10 Kohlenstoffatome enthält und ausgewählt ist aus den aliphatischen Kohlenwasserstoffgruppen, den Amino-Kohlenwasserstoffgruppen und den oxygenierten Amino-Kohlenwasserstoffgruppen.

9. Verbindungen nach Anspruch 7 der folgenden Formel (IX): 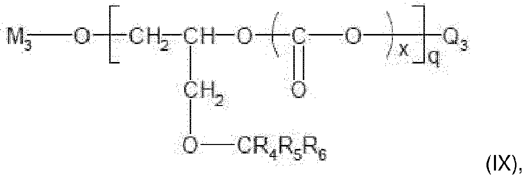 in der:
- q eine ganze Zahl größer als 1 und kleiner als 500 ist, und;
- x eine ganze Zahl gleich 0 oder 1 ist, die in der Formel (IX) gemäß jeder Gruppe der Formel (XI-a) variieren kann: 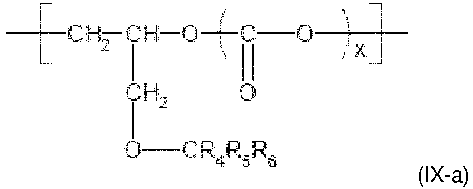 wobei x nicht immer null ist, und;
- M₃ Wasserstoff (H) ist und;
- Q₃ eine organische Gruppe darstellt, die höchstens 10 Kohlenstoffatome enthält und ausgewählt ist aus den aliphatischen Kohlenwasserstoffgruppen, den Amino-Kohlenwasserstoffgruppen und den oxygenierten Amino-Kohlenwasserstoffgruppen.

10. Verbindungen nach Anspruch 7, **dadurch gekennzeichnet, dass** Q₁ die Gruppe -CH₂-CH₂-NH₂ darstellt.

11. Verbindungen nach einem der Ansprüche 8 bis 9, **dadurch gekennzeichnet, dass** Q₃ die Gruppe -CH₂-CH₂-NH₂ darstellt.

12. Verbindungen nach einem der Ansprüche 7 oder 11 der folgenden allgemeinen Formel (V): 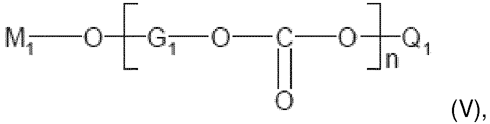 in der n eine ganze Zahl größer als 1 und unterschiedlich zu 1 ist.

13. Verbindungen nach einem der Ansprüche 7 bis 12, **dadurch gekennzeichnet, dass** R₄, R₅ und R₆ ausgewählt sind aus der Gruppe, die von Wasserstoff (H) und organischen Gruppen der Formel (VII), die wie im Anspruch 7 definiert sind, gebildet ist.

14. Zusammensetzung, die mindestens einen linearen Carbonester des α/α'-alkoxylierten Glyzerins nach einem der Ansprüche 7 bis 13 enthält.

## Claims

1. A method for synthesizing α/α'-alkoxylated glycerol linear carbonic esters wherein the following are placed in contact:
i. a quantity of at least one precursor selected from the group formed by α/α'-alkoxylated glycerol cyclic carbonates of following formula (XXII): 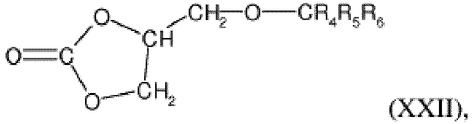 wherein:
- R₄, R₅ and R₆ are hydrogen, or
- R₄ and R₅ are hydrogens and R₆ is an alkyl or amino-alkyl group having 1 to 5 carbon atoms, or
- R₄, R₅ and R₆ are selected from the group formed by hydrogen and organic groups of following general formula (VII): 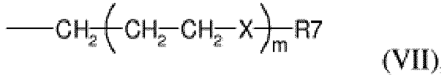 where:
• R₇ is selected from the group formed of an H and a hydrocarbon group having 1 to 6 carbon atoms;
• m is an integer between 0 and 10 inclusive; and
• X is selected from among -O- and -NH-, and ;
ii. a quantity of at least one catalyst selected from the group formed by metal oxides, metal alkoxides, Lewis acids, organometallic catalysts and mineral bases;
iii. a quantity of at least one organic initiator selected from the group formed by alcohols, polyols and amino-alcohols,
at a reaction temperature lower than 220°C,
**characterized in that** at a first step the precursor(s), catalyst(s) and organic initiator(s) are mixed and heated in a gas-tight sealed reactor until they reach the reaction temperature and a pressure called autogenous pressure higher than atmospheric pressure inside the sealed reactor, then, at the reaction temperature, at a second step gaseous composition is allowed to escape to reduce the pressure inside the reactor and the reaction temperature is maintained in the reactor for a time of more than 5 min.

2. The method according to claim 1, **characterized in that** at least one organic initiator is selected from the group formed by amino-alcohols having at least one amine selected from the group formed by primary amines, secondary amines and tertiary amines.

3. The method according to one of claims 1 or 2, **characterized in that** at least one organic initiator is triethanolamine.

4. The method according to one of claims 1 to 3, **characterized in that** at least one organic initiator is selected from the group formed by amino-alcohols having a main chain comprising 2 to 10 carbon atoms.

5. The method according to one of claims 1 to 4, **characterized in that** at least one organic initiator is selected from the group formed by 1-amino-1-cyclopentanemethanol, 4-aminocyclohexanol, amino-1-butanol, 2-amino-1-butanol, 2-amino-1-phenyl-1,3-propanediol, 3-amino-3-phenylpropan-1-ol and 2-amino-3-phenyl-1-propan-1-ol.

6. The method according to one of claims 1 to 5, **characterized in that** at least one catalyst is selected from the group formed by zinc sulfate and zinc stearate.

7. α/α'-alkoxylated linear glycerol cyclic carbonic esters of following general formula (VI): 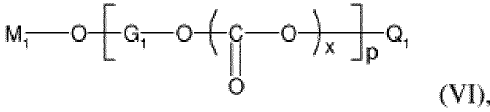 wherein:
- p is an integer higher than 1, differing from 1 and lower than 500;
- x is an integer equalling 0 or 1 and possibly varying in formula (VI) in each group of formula (VI-a): 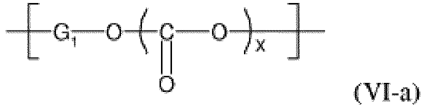 x not always being zero; and:
- M₁ is hydrogen; and
- Q₁ is an organic group having no more than 10 carbon atoms and chosen from hydrocarbon aliphatic groups, amino hydrocarbon groups and oxygenated amino hydrocarbon groups; and
- G₁ is an α/α'-alkoxylated propyl group of following general formula (II'): 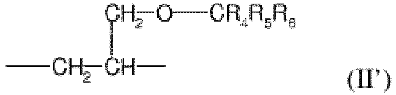 where
- R₄, R₅ and R₆ are hydrogen, or
- R₄ and R₅ are hydrogens and R₆ is an alkyl or amino-alkyl group having 1 to 5 carbon atoms, or
- R₄, R₅ and R₆ are selected from the group formed by hydrogen and organic groups of following general formula (VII): 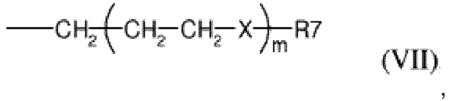 where:
• R₇ is selected from the group formed of an H and a hydrocarbon group having 1 to 6 carbon atoms;
• m is an integer between 0 and 10 inclusive; and
• X is selected from among -O- and -NH-.

8. The compounds according to claim 7 of following formula (VIII): 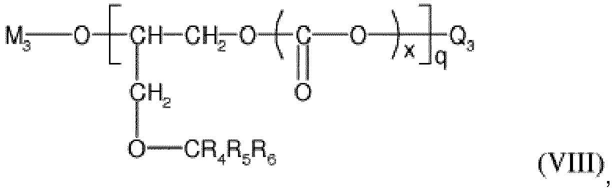 wherein:
- q is an integer higher than 1 and lower than 500; and
- x is an integer equal to 0 or 1 possibly varying in formula (VIII) in each group (VIII-a): 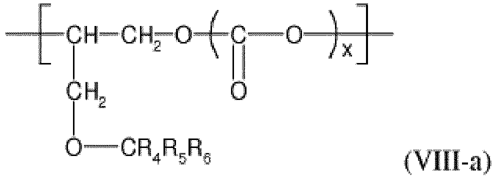 x not always being zero; and
- M₃ is hydrogen (H); and
- Q₃ is an organic group having no more than 10 carbon atoms and chosen from hydrocarbon aliphatic groups, amino hydrocarbon groups and oxygenated amino hydrocarbon groups.

9. The compounds according to claim 7 of following formula (IX): 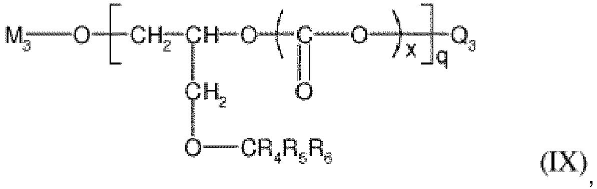 wherein:
- q is an integer higher than 1 and lower than 500; and
- x is an integer equal to 0 or 1 possibly varying in formula (IX) in each group of formula (IX-a): 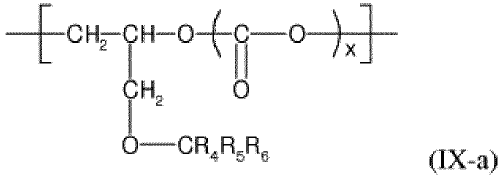 x not always being zero; and
- M₃ is hydrogen (H); and
- Q₃ is an organic group having no more than 10 carbon atoms and chosen from hydrocarbon aliphatic groups, amino hydrocarbon groups and oxygenated amino hydrocarbon groups.

10. The compounds according to claim 7, **characterized in that** Q₁ represents the group -CH₂-CH₂-NH₂.

11. The compounds according to any one of claims 8 to 9, **characterized in that** Q₃ represents the group -CH₂-CH₂-NH₂.

12. The compounds according to one of claims 7 or 11 of following general formula (V): 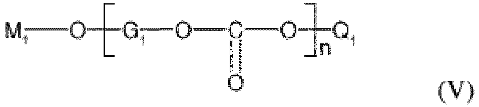 where n is an integer higher than 1 and differing from 1.

13. The compounds according to one of claims 7 to 12, **characterized in that** R₄, R₅ and R₆ are selected from the group formed by hydrogen (H) and organic groups of formula (VII) as defined in claim 7.

14. Composition comprising at least one α/α'-alkoxylated glycerol linear carbonic ester according to one of claims 7 to 13.
